# EUROPEAN PATENT APPLICATION

(11) **EP 4 183 406 A1**
(43) Date of publication of application: **24.05.2023**
(21) Application number: 21383046.6
(22) Date of filing: 19.11.2021
(51) Int. Cl.: A61K 38/10, A61K 38/16, A61K 38/17, C07K 14/47

(54) **IN VITRO METHODS AND KITS FOR RESPIRATORY TRACT VIRAL DISEASES**

(71) Applicant: Fundación para el Fomento de la Investigación Sanitaria y Biomédica de la Comunitat Valenciana (FISABIO), 46020 Valencia (ES)
(72) Inventor: Mira Obrador, Alejandro, 46185 Pobla de Vallbona (Valencia) (ES); Ferrer García, María de los Desamparados, 46006 Valencia (ES); Corell Escuin, Paula, 46001 Valencia (ES); López Labrador, Francisco Javier, 46019 Valencia (ES)
(74) Representative: Manuel Illescas y Asociados, S.L.

(57) **Abstract**

A method and a kit for determining *in vitro* the resistance of an individual to suffer from symptoms of a virus affecting the respiratory tract and, more in particular, the symptoms of a disease caused by a virus affecting the respiratory tract selected from the group consisting of an influenza virus; a coronavirus; a syncytial virus (RSV); a rhinovirus (RVs); a parainfluenza virus (PIV); an adenovirus (ADV); or a morbillivirus and, consequently, to suffer from said viral disease. Also described are methods and kits for selecting individuals in need of prophylactic measures against said virus affecting the respiratory tract.

## Description

### TECHNICAL FIELD OF THE INVENTION

The present invention relates to an *in vitro* method for determining the level of resistance of an individual to develop symptoms of a viral disease of the respiratory tract and, more in particular, the symptoms of a disease caused by a virus affecting the respiratory tract selected from the group consisting of an influenza virus; a coronavirus; a syncytial virus (RSV); a rhinovirus (RVs); a parainfluenza virus (PIV); an adenovirus (ADV); or a morbillivirus and, consequently, to suffer from said viral disease. Additionally, present invention refers to methods and kits for selecting individuals in need of prophylactic measures against said virus affecting the respiratory tract.

### BACKGROUND OF THE INVENTION

Viruses, and in particular coronavirus and influenza virus, are a major cause of respiratory diseases, causing annual epidemics and even pandemics with a severe impact on public health, accounting for hundreds of thousands to millions of deaths worldwide.

Due to the burden of diseases associated with virus of the respiratory tract several approaches have been developed to fight against said infectious agents. In developed countries, annual influenza vaccination campaigns have been established for persons at greater risk. The COVID-19 pandemic has also mobilized worldwide vaccination campaigns to avoid deaths and healthcare collapse. But a vaccine developed one year may not be effective the following year due to the frequent and rapid mutations (changes in its antigens) of the virus and the varying dominance of the various strains of the virus itself. Accordingly, determining the groups of individuals who are more prone to suffer from said viral diseases would help to establish vaccination plans for each of said viral diseases, even in a yearly basis.

It is therefore necessary to evaluate the risk of suffering from acute symptoms of viral respiratory infections with the aim to discern the individuals of higher risk for developing severe complication from others who can be catalogued as resistant to said infection or as asymptomatic, i.e. individuals who have never suffered from flu or influenza symptoms.

### BRIEF DESCRIPTION OF THE INVENTION

The present invention describes an *in vitro* method for determining the level of resistance of a healthy individual to suffer from symptoms of a virus affecting the respiratory tract, wherein said method comprises determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in an *ex vivo* biological sample selected from the group consisting of a nasopharyngeal sample, a blood sample, a plasma sample, a urine sample, a human milk sample, a sweat sample and a teardrop sample; wherein the higher the concentration of said peptides in the sample is, the greater the resistance of said individual is to suffer from symptoms of said virus.

The present invention further relates to a kit for conducting a method for determining the level of resistance of a healthy individual to suffer from symptoms of a virus affecting the respiratory tract, wherein said kit comprises:
- means for storing an ex *vivo* biological sample of said healthy individual selected from the group consisting of a nasopharyngeal sample, a blood sample, a plasma sample, a urine sample, a human milk sample, a sweat sample and a teardrop sample;
- means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in said stored ex *vivo* biological sample.

Present invention also refers to an *in vitro* method of selecting healthy individuals in need of prophylactic measures against a disease caused by a virus affecting the respiratory tract, wherein said method comprises:
- determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in an ex *vivo* biological sample of a healthy individual selected from the group consisting of a saliva sample, a nasopharyngeal sample, a blood sample, a plasma sample, a urine sample, a human milk sample, a sweat sample and a teardrop sample;
- comparing said total concentration of peptides in said ex *vivo* biological sample with a threshold value; and
- selecting said healthy individual for prophylactic measures against said disease if the value of said total concentration of said peptides in the ex *vivo* biological sample of said individual is lower than said threshold value.

Finally, present invention also refers to a kit for conducting the method of selecting healthy individuals in need of prophylactic measures against a disease caused by a virus affecting the respiratory tract, according to any of claims 2 to 9, wherein said kit comprises:
means for storing an ex *vivo* biological sample of a healthy individual selected from the group consisting of a saliva sample, a nasopharyngeal sample, a blood sample, a plasma sample, a urine sample, a human milk sample, a sweat sample and a teardrop sample;
- means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in said stored ex *vivo* biological sample;
- means and/or instructions for identifying values of said total concentration of peptides which are lower of a threshold value.

### BRIEF DESCRIPTION OF THE FIGURES

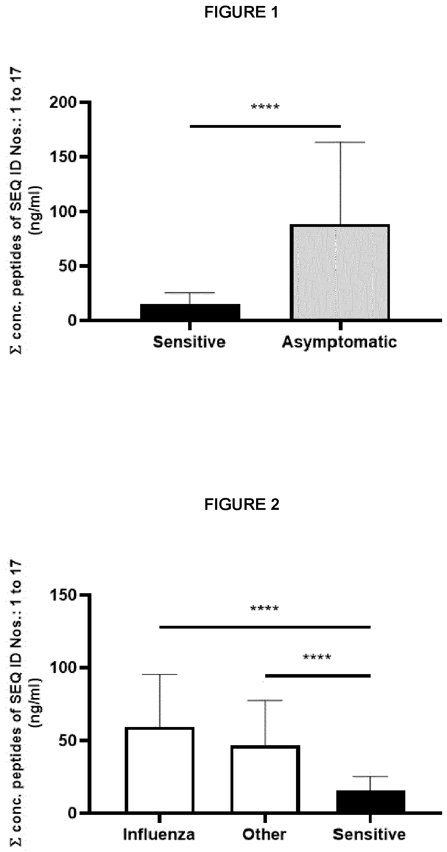

**Figure 1** Mean and standard deviation of total concentration of peptides with sequence SEQ ID No.: 17 and of any fragment thereof (of SEQ ID Nos.: 1 to 16) in ng/ml in nasopharyngeal swab human samples from healthy individuals who are sensitive or prone to having respiratory tract infection symptoms ("Sensitive", n=29) compared to healthy individuals who never suffered from influenza virus symptoms ("Asymptomatic", n=30). The difference in between these two groups is statistically significant (p-value < 0.0001). Measurements were taken by immunoassay test (ELISA), according to the method of example 1.
**Figure 2****.** Mean and standard deviation of total concentration of peptides with sequence SEQ ID No.: 17 and of any fragment thereof (of SEQ ID Nos.: 1 to 16), expressed in ng/ml in nasopharyngeal swab samples from inpatients hospitalized for respiratory tract pathology and positive for influenza A virus by nasopharyngeal PCR test ("Influenza", n=30), inpatients hospitalized for respiratory tract pathology and positive by nasopharyngeal PCR test for other respiratory viruses such as respiratory syncytial virus (RSV), metapneumovirus, parainfluenza virus (PIV), rhinovirus (RV), adenovirus (ADV), common coronavirus or bocavirus ("Other", n=31), both compared to values from healthy individuals who are sensitive or prone to having respiratory tract infection symptoms ("Sensitive", n=29), p-values < 0.0001. Measurements were performed by immunoassay test (ELISA), according to the method in example 1.

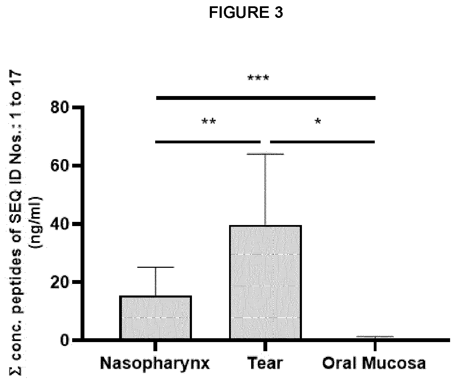

**Figure 3****.** Mean and standard deviation of total concentration of peptides with sequence SEQ ID No.: 17 and of any fragment thereof (of SEQ ID Nos.: 1 to 16), expressed in ng/ml in nasopharyngeal swab samples ("Nasopharynx", n=30), teardrop samples ("Tear", n=5) and oral mucosa samples ("Oral Mucosa", n=3) from healthy individuals who are sensitive of having respiratory tract infection symptoms. The difference is statistically significant, *** p-value = 0.0004, ** p-value = 0.0039, * p-value = 0,0357. Measurements were obtained by immunoassay test (ELISA), according to the method of example 1.

### DESCRIPTION OF THE INVENTION

Dermcidin is a protein of human origin which, once secreted, is processed (i.e. suffers an enzymatic fragmentation) to give rise to different fragments or smaller peptides of different biological activity. The sequence of dermcidin consists of 110 aminoacids of which, starting at the N-terminus end, the first 19 aminoacids correspond to the signal peptide, the next 43 aminoacids make up the prodomain and the next 48 aminoacids are called the antimicrobial domain, or AMP. The name AMP refers to the fact that the prior art has described antimicrobial effects derived from peptides with this sequence (patent publication number DE10129983 A1).

For the purposes of the present invention, the sequence of dermcidin (isoform 1, NCBI Reference Sequence: NP_444513.1) is SEQ ID No.: 17. Accordingly, the term "dermcidin", refers, for the purposes of present invention, to a peptide of sequence SEQ ID No.: 17.

On the other hand, for the purposes of the present invention, any of the peptides having (consisting of) the aminoacidic sequences of the peptide fragments which are result of the enzymatic processing of dermcidin occurring in the human organism, are referred to as "dermcidin peptides" or "dermcidin fragments". Said dermcidin fragments may be of natural origin (i.e. being result of said natural enzymatic processing of dermcidin in the human organism) or may be obtained by synthesis. The aminoacid sequences of the protein dermcidin and these "dermcidin fragments" are listed in Table 1:

**Table 1. Dermcidin and fragments thereof**

| Table 1 | | |
|---|---|---|
| LEK-42 | SEQ ID No.: 1 | LEKGLDGAKK AVGGLGKLGK DAVEDLESVG KGAVHDVKDV LD |
| LEK-45 | SEQ ID No.: 2 | LEKGLDGAKK AVGGLGKLGK DAVEDLESVG KGAVHDVKDV LDSVL |
| SSL-45 | SEQ ID No.: 3 | SSLLEKGLDG AKKAVGGLGK LGKDAVEDLE SVGKGAVHDV KDVLD |
| DCD | SEQ ID No.: 4 | SSLLEKGLDG AKKAVGGLGK LGKDAVEDLE SVGKGAVHDV KDVLDSV |
| DCD-1L | SEQ ID No.: 5 | SSLLEKGLDG AKKAVGGLGK LGKDAVEDLE SVGKGAVHDV KDVLDSVL |
| SSL-46 | SEQ ID No.: 6 | SSLLEKGLDG AKKAVGGLGK LGKDAVEDLE SVGKGAVHDV KDVLDS |
| LEK-44 | SEQ ID No.: 7 | LEKGLDGAKK AVGGLGKLGK DAVEDLESVG KGAVHDVKDV LDSV |
| LEK-43 | SEQ ID No.: 8 | LEKGLDGAKK AVGGLGKLGK DAVEDLESVG KGAVHDVKDV LDS |
| SSL-29 | SEQ ID No.: 9 | SSLLEKGLDG AKKAVGGLGK LGKDAVEDL |
| SSL-25 | SEQ ID No.: 10 | SSLLEKGLDG AKKAVGGLGK LGKDA |
| LEK-41 | SEQ ID No.: 11 | LEKGLDGAKK AVGGLGKLGK DAVEDLESVG KGAVHDVKDV L |
| LEK-26 | SEQ ID No.: 12 | LEKGLDGAKKAVGGLGKLGK DAVEDL |
| LEK-24 | SEQ ID No.: 13 | LEKGLDGAKKAVGGLGKLGK DAVE |
| YDP-42 | SEQ ID No.: 14 | YDPEAASAPG SGNPCHEASA AQKENAGEDP GLARQAPKPR KQ |
| Y-P30 | SEQ ID No.: 15 | YDPEAASAPG SGNPCHEASA AQKENAGEDP |
| PIF | SEQ ID No.: 16 | YDPEAASAPG SGNPCHEAS A |
| Dermcidin (NCBI Ref. Seq: NP_444513*1 | SEQ ID No.: 17 | MRFMTLLFLT ALAGALVCAY DPEAASAPGS GNPCHEASAA QKENAGEDPG LARQAPKPRK QRSSLLEKGL DGAKKAVGGL GKLGKDAVED LESVGKGAVH DVKDVLDSVL |

Accordingly, for the purposes of the present invention the term "dermcidin" refers to a peptide of sequence SEQ ID No.:17, i.e., a peptide consisting of sequence SEQ ID No.: 17.

On the other hand, the term "dermcidin fragment" refers to a peptide consisting of, or comprising, any of the aminoacidic sequences SEQ ID Nos.: 1, 2, 3, 4, 5, 6, 7, 8, 9, 10, 11, 12, 13, 14, 15 and 16 disclosed in table 1.

Additionally, for the purpose of present invention, the term "dermcidin peptides" refers to any peptide of sequences SEQ ID Nos.: 1, 2, 3, 4, 5, 6, 7, 8, 9, 10, 11, 12, 13, 14, 15, 16 and 17 disclosed in table 1, i.e., it refers to both the complete dermcidin peptide (SEQ ID No.: 17) and any fragment thereof (SEQ ID No.: 1 to 16).

Thus, the term "a peptide of sequence SEQ ID Nos.: 17 or any of the fragments thereof" or the term "a peptide of any of sequences SEQ ID Nos.: 1 to 17", refer to any peptide consisting of one of the aminoacidic sequences SEQ ID Nos.: 1 to 17, as disclosed in table 1 herein.

Finally, the term "dermcidin derivative" refers to a peptide having a sequence in which at least one aminoacid is different from the aminoacid present in the natural occurring sequence, because said aminoacid has been artificially modified or replaced; or additionally, to a peptide having at least 80%, preferably at least 90%, and more preferably at least 95% sequence identity, with any of the aminoacidic sequences SEQ ID Nos.: 1, 2, 3, 4, 5, 6, 7, 8, 9, 10, 11, 12, 13, 14, 15, 16 and 17 disclosed in table 1 herein. In a preferred embodiment of the invention the dermcidin derivative is a peptide comprising or consisting of a sequence selected from the group consisting of SEQ ID Nos.: 1, 2, 3, 4, 5, 6, 7, 8, 9, 10, 11, 12, 13, 14, 15, 16 and 17, in which at least one aminoacid is modified. Said modification may be artificial or naturally occurring. In a more preferred embodiment of the invention the dermcidin derivative is a peptide comprising or consisting of a sequence selected from the group consisting of SEQ ID Nos.: 1, 2, 3, 4, 5, 6, 7, 8, 9, 10, 11, 12, 13, 14, 15, 16 and 17, in which at least one aminoacid is acetylated, methylated, formylated, amidated or phosphorylated. In an even more preferred embodiment, said peptide is a derivative in which at least one aminoacid is acetylated.

Thus, in a preferred embodiment of the invention the dermcidin derivative is a peptide comprising or consisting of any of sequences SEQ ID Nos.: 1, 2, 7, 8, 11, 12 and 13, in which the lysine in position 3 is acetylated, wherein said dermcidin derivatives comprise or consist, respectively, of a sequence SEQ ID Nos.: 19, 20, 25, 26, 29, 30 and 31. In another more preferred embodiment of the invention the dermcidin derivative is a peptide comprising or consisting of a sequence selected from the group consisting of SEQ ID Nos.: 3, 4, 5, 6, 9 and 10, in which the lysine in position 6 is acetylated, wherein said dermcidin derivatives comprise or consist, respectively, of a sequence SEQ ID Nos.: 21, 22, 23, 24, 27 and 28. In another more preferred embodiment the dermcidin derivative is a peptide comprising or consisting of a sequence SEQ ID No: 17, in which the lysine in position 68 is acetylated wherein said dermcidin derivative comprises or consists of SEQ ID No. 32. Said derivative dermcidin peptides of SEQ ID Nos.: 19 to 32 are included in the following table 1.2:

**Table 1.2: Acetylated derivative dermcidin peptides. Acetylated aminoacid is marked with an asterisk * (i.e. acetylated lysine is K*).**

| Table 1.2 | | |
|---|---|---|
| SEQ ID No. 19 | Derivative of SEQ ID No. :1 with acetylated lysine in position 3 | LEK*GLDGAKK AVGGLGKLGK DAVEDLESVG KGAVHDVKDV LD |
| SEQ ID No.: 20 | Derivative of SEQ ID No. :2 with acetylated lysine in position 3 | LEK*GLDGAKK AVGGLGKLGK DAVEDLESVG KGAVHDVKDV LDSVL |
| SEQ ID No.: 21 | Derivative of SEQ ID No. :3 with acetylated lysine in position 6 | SSLLEK*GLDG AKKAVGGLGK LGKDAVEDLE SVGKGAVHDV KDVLD |
| SEQ ID No.: 22 | Derivative of SEQ ID No. :4 with acetylated lysine in position 6 | SSLLEK*GLDG AKKAVGGLGK LGKDAVEDLE SVGKGAVHDV KDVLDSV |
| SEQ ID No.: 23 | Derivative of SEQ ID No. :5 with acetylated lysine in position 6 | SSLLEK*GLDG AKKAVGGLGK LGKDAVEDLE SVGKGAVHDV KDVLDSVL |
| SEQ ID No.: 24 | Derivative of SEQ ID No. :6 with acetylated lysine in position 6 | SSLLEK*GLDG AKKAVGGLGK LGKDAVEDLE SVGKGAVHDV KDVLDS |
| SEQ ID No.: 25 | Derivative of SEQ ID No. :7 with acetylated lysine in position 3 | LEK*GLDGAKK AVGGLGKLGK DAVEDLESVG KGAVHDVKDV LDSV |
| SEQ ID No.: 26 | Derivative of SEQ ID No. :8 with acetylated lysine in position 3 | LEK*GLDGAKK AVGGLGKLGK DAVEDLESVG KGAVHDVKDV LDS |
| SEQ ID No.: 27 | Derivative of SEQ ID No. :9 with acetylated lysine in position 6 | SSLLEK*GLDG AKKAVGGLGK LGKDAVEDL |
| SEQ ID No.: 28 | Derivative of SEQ ID No. :10 with acetylated lysine in position 6 | SSLLEK*GLDG AKKAVGGLGK LGKDA |
| SEQ ID No.: 29 | Derivative of SEQ ID No. :11 with acetylated lysine in position 3 | LEK*GLDGAKK AVGGLGKLGK DAVEDLESVG KGAVHDVKDV L |
| SEQ ID No.: 30 | Derivative of SEQ ID No. :12 with acetylated lysine in position 3 | LEK*GLDGAKK AVGGLGKLGK DAVEDL |

| Table 1.2 (cont.) | | |
|---|---|---|
| SEQ ID No.: 31 | Derivative of SEQ ID No. :13 with acetylated lysine in position 3 | LEK*GLDGAKK AVGGLGKLGK DAVE |
| SEQ ID No.: 32 | Derivative of SEQ ID No. :17 with acetylated lysine in position 68 | MRFMTLLFLT ALAGALVCAY DPEAASAPGS GNPCHEASAA QKENAGEDPG LARQAPKPRK QRSSLLEK*GL DGAKKAVGGL GKLGKDAVED LESVGKGAVH DVKDVLDSVL |

The different abbreviations and sequences ID numbers of the peptides dermcidin and fragments thereof are defined, for the purposes of the present invention in the above referred table 1. Accordingly, even though in some documents of the state of the art, the peptide dermcidin (herein disclosed as SEQ ID No: 17) is commonly abbreviated as DCD, for the purposes of the present disclosure, the abbreviation DCD is only used in the present disclosure for the peptide of sequence SEQ ID No.: 4. In the same manner, even though in some documents of the state of the art the peptide of SEQ ID No: 4 is designated with the abbreviation DCD-1, for the purposes of the present invention the dermcidin fragment of sequence SEQ ID No.: 4 is abbreviated in the present disclosure as DCD, whereas the dermcidin fragment of sequence SEQ ID No: 5 is abbreviated in the present disclosure as DCD-1L.

Dermcidin was thought to be present only in tissues such as sweat, placenta, blood or human milk. In the present invention, dermcidin is detected for the first time in human nasopharyngeal exudate and teardrops, as described herein in example 1.

In fact, present invention shows that, surprisingly, as seen in example 1 and figure 1 disclosed herein, the production of dermcidin (SEQ ID No.: 17) and, consequently, of any of the fragments thereof of any of sequences SEQ ID Nos.: 1, 2, 3, 4, 5, 6, 7, 8, 9, 10, 11, 12, 13, 14, 15 or 16, as disclosed in table 1 (i.e. the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16), as well as its presence in ex *vivo* biological samples, is significantly higher in healthy individuals who have never suffered from flu (influenza virus) symptoms, when compared to the values of healthy individuals sensitive or prone to having respiratory tract viral infections.

According to present invention, the term "individuals sensitive or prone to having respiratory tract viral infections" or "individuals sensitive or prone to suffer from symptoms of a virus affecting the respiratory tract" refers to individuals that have been previously hospitalized for respiratory tract pathology and positive by nasopharyngeal PCR test for the following respiratory viruses: influenza A virus, respiratory syncytial virus (RSV), metapneumovirus, parainfluenza virus (PIV), rhinovirus (RV), adenovirus (ADV), common coronavirus or bocavirus.

Additionally, according to present invention the term "individuals who have never suffered from flu symptoms" or the term "individuals who have never suffered from respiratory tract infection symptoms" refers to healthy individuals who have never suffered from influenza or flu symptoms (i.e. do not remember having suffered from flu symptoms), wherein said influenza symptoms are, as defined by the World Health Organization: sudden onset of fever, cough (usually dry), headache, muscle and joint pain, severe malaise (feeling unwell), sore throat and a runny nose.

The significant difference found in the levels of production of dermcidin (total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16), as well as the significant difference found in the levels of said peptides present in ex *vivo* biological samples of individuals who have never suffered from flu symptoms, when compared to those prone to suffer from symptoms of a virus affecting the respiratory tract, allows determining whether a certain individual (or test individual) is more or less prone to suffer from symptoms of a virus affecting the respiratory tract.

Additionally, said significant difference in the levels of dermcidin (total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16) in ex *vivo* biological samples of individuals who have never suffered from flu symptoms and of individuals prone to suffer from symptoms of a virus affecting the respiratory tract, allows determining a concentration of dermcidin (total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16), or threshold value, below which it may be considered that a certain individual is in a significant risk to be infected and suffer from symptoms of a virus affecting the respiratory tract.

For the purposes of present invention said threshold value is, accordingly, the average value of the concentration of dermcidin (total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16) obtained in an *ex vivo* biological sample from a control group of individuals who have never suffered from flu symptoms, as defined herein, by using the same type of ex *vivo* biological sample and the same method to determine said dermcidin concentration (total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16) than the ex *vivo* biological sample and the method used to evaluate the risk of a particular individual to suffer from symptoms of a virus affecting the respiratory tract.

Thus, the invention discloses an *in vitro* method for determining the level of resistance of a healthy individual to suffer from symptoms of a virus affecting the respiratory tract, wherein said method comprises determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, as disclosed in table 1 herein, in an ex *vivo* biological sample selected from the group consisting of a nasopharyngeal sample, a blood sample, a plasma sample, a urine sample, a human milk sample, a sweat sample and a teardrop sample; wherein the higher the concentration of said peptides in the sample is, the greater the resistance of said healthy individual is to suffer from symptoms of said virus.

For the purposes of the present invention, the term "comprises" indicates that it includes a group of features but does not exclude the presence of other additional features, provided that the presence of the other additional features does not render the claim impracticable. In addition, the terms "consists of", "contains", "includes", "has", "encompasses" and synonyms of such terms are to be construed in the same manner as the term "comprises".

Additionally, for the purposes of the present invention, the term "comprises" may be replaced by any of the terms "includes", "consists of", "substantially consists of" or "substantially consist in". Thus, where the term "comprises" refers to a group of technical features A, B and C, it should be construed as including, additionally, other technical features in addition to technical features A, B and C, provided that the presence of the other features does not render the claim impracticable; but may also be construed as comprising only such features A, B and C or substantially such features A, B and C, and therefore the term "comprises" referring to a group comprising features A, B and C is to be construed as including a group consisting of features A, B and C, or consisting substantially of features A, B and C.

For the purposes of present invention the term "total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16" is also equivalent to the term "concentration of dermcidin and of any fragment thereof".

For the purposes of the present invention the concentration of dermcidin, i.e. the total concentration of the peptide of SEQ ID No.: 17, or of any fragment thereof of SEQ ID Nos.: 1 to 16; which is comprised in said sample, may be measured directly by determining the quantity of said peptides present in the ex vivo biological sample or, indirectly, by methods, such as PCR assays, which measure the levels of gene expression of dermcidin, i.e. methods which measure the levels of gene expression encoding for the peptide of SEQ ID No.: 17 (and consequently that of fragments of SEQ ID No.: 1 to 16 produced by fragmentation of said peptide of SEQ ID No.: 17).

For the purposes of the present invention the terms "viruses affecting the respiratory tract", "respiratory virus", "viral diseases of the respiratory tract", or the like, refer to diseases that present adverse symptoms in the respiratory tract and are caused by a virus.

In an embodiment of present invention, the virus affecting the respiratory tract is selected, without limitation, from the group consisting of: an influenza virus; a coronavirus; a bocavirus; a syncytial virus (RSV); a metapneumovirus; a rhinovirus (RVs); a parainfluenza virus (PIV); an adenovirus (ADV); and a morbillivirus, such as a measles virus.

Preferably, said virus affecting the respiratory tract is selected from the group consisting of an influenza virus; a coronavirus, selected from the group consisting of human coronavirus OC43 (the main cause of common cold), SARS, MERS and SARS-COV-2; a syncytial virus (RSV); a rhinovirus (RVs); a parainfluenza virus (PIV); an adenovirus (ADV); a metapneumovirus; a bocavirus; and a morbillivirus such as the measles virus.

Preferably, the coronavirus is selected from the group consisting of a coronavirus HcoV-OC43, SARS, MERS or SARS-CoV-2.

Accordingly, most preferably the virus affecting the respiratory tract is selected from the group consisting of an influenza virus (flu virus), a coronavirus HcoV-OC43 (common cold virus), SARS, MERS, SARS-CoV-2, a syncytial virus (RSV); a rhinovirus (RVs); a parainfluenza virus (PIV); a metapneumovirus; a bocavirus; an adenovirus (ADV); and a morbillivirus.

Even more preferably, the virus affecting the respiratory tract is selected from the group consisting of an influenza virus (flu virus), a coronavirus HcoV-OC43 (common cold virus), SARS, MERS and SARS-CoV-2. Yet even more preferably, the virus affecting the respiratory tract is SARS-CoV-2 or an influenza virus.

In a preferred embodiment the virus affecting the respiratory tract is an influenza virus, a coronavirus or a morbillivirus. In a more preferred embodiment, the coronavirus is HcoV-OC43 or SARS-CoV-2, the morbillivirus is a measles virus and the influenza virus comprises an H1N1 subtype and/or an H3N2 subtype.

Additionally, the examples included in present invention could confirm that the levels of dermicidin (measured as the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16) may also be measured in other human fluids, such as tears.

Accordingly, in a preferred embodiment said ex *vivo* biological sample is a nasopharyngeal sample or a teardrop sample.

In a more preferred embodiment, said ex *vivo* sample is a nasopharyngeal sample.

In an embodiment of present invention, the determination of the peptide's concentration is performed by any method capable of measuring the concentration of a protein or a peptide comprising any of sequences SEQ ID Nos. 1 to 17 in a biological sample, and in particular a method selected from the group consisting of mass spectrometry (MS), liquid chromatography-mass spectrometry (LC-MS), high pressure liquid chromatography (HLPC), immunoassay and polymerase chain reaction (PCR)-based assays.

Preferably, the determination of the peptide's concentration is performed by immunoassay, measuring the levels of dermcidin produced (total concentration of peptides comprising any of sequences SEQ ID Nos.: 1 to 17) and, accordingly, the method of the invention determines the total quantity of the peptides of SEQ ID Nos.: 1 to 17 present in an ex vivo biological sample.

For the purposes of present invention the term immunoassay refers to a method to determine the concentration of peptides having a specific sequence, in a biological sample, which include the use of antibodies specific for binding to said peptides.

In a preferred embodiment of the invention said immunoassay is an ELISA test, an ELISPOT test, a bead-based multiplex immunoassay (Immunoflex), a Western Blot assay, a flow cytometry assay, a fluorescence-activated cell sorting assay (FACS), a radioimmunoassay, a colorimetric or a fluorometric diagnostic strip comprising antibodies, colorimetric nanoparticles comprising antibodies, a chemiluminescent assay, an immunocytochemistry assay (with or without paraffin protocol), an immunofluorescence assay, an immunohistochemical assay, among others.

More preferably said method is performed by immunoassay comprising antibodies specific for binding to a peptide comprising any of sequences SEQ ID Nos. 1 to 17, as disclosed in table 1 herein. Said antibodies may be monoclonal antibodies or polyclonal antibodies. Preferably said antibodies have an Ig, an IgG, an IgG1 or an IgM isotype. In some preferred embodiments said antibodies are unconjugated, in other preferred embodiments said antibodies are conjugated or tagged with HRP (horse radish peroxidase), FITC (fluorescein isothiocyanate), Biotin or with cyanine dyes such as Cy3, Cy5, Cy5.5 or Cy7.

Examples of antibodies specific for binding to a peptide comprising any of sequences SEQ ID Nos. 1 to 17 are those included in table 1.3 included herein:

**Table 1.3: Examples of antibodies to be used in the methods and kits of the invention (WB: Western Blot, RIA: radioimmunoassay, IF: Immunofluorescence, IHC: Immunohistochemistry, IHC-p: Immunohistochemistry-paraffin protocol; IHC-fr: Immunohistochemistry frozen sections protocol; ICC: Immunocytochemistry; Flo: Flow cytometry; IA: Immunoassays; ELISA: Enzyme-linked immunosorbent assay; CLIA: Chemiluminescent immunoassay; ELISPOT: Enzyme-linked immunospot assay; FACS: Fluorescence-activated cell sorting; IP: Immunoprecipitation; Conj.: conjugated; Unconj.: unconjugated; Mono.: monoclonal, Poly.: polyclonal).**

| Source | Catalog No. | Purity | Host | React. | Iso. | Method | Clone/ Reg. | Conj./ Tag | Clon |
|---|---|---|---|---|---|---|---|---|---|
| LifeSpan BioSci. | LS-B6540 | Tissue culture supernatant | Mouse | Human | IgM | IHC, IHC-p, WB | G-81 | Unconj | Mono. |
| LifeSpan BioSci. | LS-C167290 | | Rabbit | Human | | Flo, IHC, IHC-p, WB | aa 74-103 | Unconj | Poly. |
| LifeSpan BioSci. | LS-C128574 | Antiserum | Rabbit | Human, Monkey, Rat | | ELISA, WB | aa 96-110 | Unconj | Poly. |
| LifeSpan BioSci. | LS-C128573 | Antiserum | Rabbit | Rat, Human | | ELISA, RIA | aa 60-88 | Unconj | Poly. |
| LifeSpan BioSci. | LS-C346322 | Affinity purified | Rabbit | Mouse, Human | IgG | IF, IHC, WB | | Unconj | Poly. |
| LifeSpan BioSci. | LS-C765989 | Affinity purified | Rabbit | Mouse, Human | IgG | WB, IF | | Unconj | Poly. |
| LifeSpan BioSci. | LS-C803620 | Peptide affinity chromatogra phy | Rabbit | Human | IgG | ELISA, WB | | Unconj | Poly. |
| LifeSpan BioSci. | LS-C317136 | Caprylic acid and ammonium sulfate precipitation | Rabbit | Human | IgG | ELISA, WB | | HRP | Poly. |
| LifeSpan BioSci. | LS-C317131 | Caprylic acid and ammonium sulfate precipitation | Rabbit | Human | IgG | ELISA, WB | | FITC | Poly. |
| LifeSpan BioSci. | LS-C317135 | Caprylic acid and ammonium sulfate precipitation | Rabbit | Human | IgG | ELISA, WB | | Biotin | Poly. |
| LifeSpan BioSci. | LS-C285486 | Caprylic acid and ammonium sulfate precipitation | Rabbit | Human | IgG | ELISA, WB | | Unconj | Poly. |
| LifeSpan BioSci. | LS-C754340 | Immunogen affinity purified to greater than 95% as determined by SDS-PAGE | Rabbit | Human | IgG | ELISA, IHC | | Unconj. | Poly. |
| LifeSpan BioSci. | LS-C482792 | Immunoaffinit y purified | Rabbit | Human | IgG | ICC, IF, WB | | Unconj. | Poly. |
| LifeSpan BioSci. | LS-C796065, LS-C792911 | Affinity chromatogra phy | Mouse | Human | IgG1 | WB | OTI3F 5, aa 20-110 | Unconj. | Mono. |
| LifeSpan BioSci. | LS-B15625 | Tissue culture supernatant | Mouse | Human | IgM | ELISA, IHC, IHC-p, WB | GF1 | Unconj. | Mono. |
| Proteintec h Group Inc | 11985-1-AP | Antigen affinity purification | Rabbit | Human | IgG | ELISA, IHC | | Unconj. | Poly. |
| MyBioSou rce.com | MBS8245 311 | Affinity chromatogra phy | Rabbit | Human | | WB, IF, ICC | | Unconj. | Poly. |
| MyBioSou rce.com | MBS2102 136 | | Rabbit | Human | | ELISA, CLIA, ELISPOT, Luminex | | Unconj. | Poly. |
| MyBioSou rce.com | MBS2003 527 | Affinity chromatogra phy | Rabbit | Human | IgG | ELISA, IHC, ICC, WB | | Unconj. | Poly. |
| MyBioSou rce.com | MBS1488 570 | Caprylic Acid Ammonium Sulfate Precipitation Purified | Rabbit | Human | IgG | ELISA, WB | | Biotin | Poly. |
| MyBioSou rce.com | MBS5842 44 | Tissue culture supernatant | Mouse | Human | IgM | WB, IA | G-81 | Unconj. | Mono. |
| MyBioSou rce.com | MBS9201 083 | Purified | Rabbit | Human | Ig | ELISA, WB, IHC, Flo/FACS | aa 74-103 | Unconj. | Poly. |
| MyBioSou rce.com | MBS9126 143 | Affinity Purification | Rabbit | Human | IgG | WB, IHC, IF | | Unconj. | Poly. |
| MyBioSou rce.com | MBS9430 229 | Antigen affinity purification | Rabbit | Human | IgG | IHC | | Unconj. | Poly. |
| MyBioSou rce.com | MBS8563 071 | Purified By Antigen Affinity Column | Rabbit | Human | IgG | ELISA, IHC | | Unconj. | Poly. |
| MyBioSou rce.com | MBS2554 200 | Affinity Purification | Rabbit | Mouse, Human | IgG | WB, IF | | Unconj. | Poly. |
| BosterBio | A01446 | Affinity Purification | Rabbit | Mouse, Human | IgG | WB, IF | | Unconj . | Poly. |
| Novus Biological s | NBP2-56558 | Affinity Purification | Rabbit | Human | IgG | IHC, IHC-P | | Unconj . | Poly. |
| Novus Biological s | NBP2-92673-0.1ml | Affinity Purification | Rabbit | Mouse, Human | IgG | WB, IF, IHC | aa 20-110 | Unconj . | Poly. |
| Novus Biological s | NBP1-49620-1ml | Tissue culture supernatant | Mouse | Human | IgM | WB, IHC, IHC-p, IF, ICC, IA | G-81 | Unconj . | Mono. |
| Biorbyt | orb36923 | Affinity Purification | Rabbit | Human | IgG | ELISA, FACS, IHC-p, WB | aa 74-110 | Unconj . | Poly. |
| Biorbyt | orb15657 4 | Affinity purified by Protein A | Rabbit | Human | IgG | WB, ELISA, ICC, IF, IHC-p | aa 73-99 | Unconj ., HRP | Poly. |
| Biorbyt | orb24157 9 | >95%, Protein G purified | Rabbit | Human | IgG | ELISA, IHC-p | aa 20-110 | Unconj ., HRP | Poly. |
| Biorbyt | orb34126 3 | immunogen affinity chromatogra phy | Rabbit | Human | IgG | WB, ICC, IF | full length | Unconj ., HRP | Poly. |
| Biorbyt | orb64925 3 | Affinity Purification | Rabbit | Mouse, Human | IgG | WB, IF | full length | Unconj . | Poly. |
| Biorbyt | orb63834 5 | Affinity Purification | Rabbit | Rat, Human | IgG | ELISA, IHC-p, WB | | Unconj . | Poly. |
| Affinity Bioscienc es | DF13367 | Affinity chromatogra phy | Rabbit | Rat, Human | IgG | WB, IHC, ELISA | | Unconj . | Poly. |
| Affinity Bioscienc es | DF13367 | Affinity chromatogra phy | Rabbit | Rat, Human | IgG | WB, IHC, ELISA | | Unconj . | Poly. |
| antibodies -online | ABIN8708 18 | | Rabbit | Human | | ELISA, RIA | aa 66-81 | Unconj . | Poly. |
| antibodies -online | ABIN8708 10 | | Rabbit | Human | | ELISA, WB | aa 96-110 | Unconj . | Poly. |
| antibodies -online | ABIN8708 11 | | Rabbit | Human | | ELISA, WB | aa 96-110 | Unconj . | Poly. |
| antibodies -online | ABIN8708 19 | | Rabbit | Human | | ELISA, RIA | aa 66-80 | Unconj . | Poly. |
| antibodies -online | ABIN2191 909 | | Mouse | Human | | IA, IHC-p, WB | G-81 | Unconj . | Mono. |
| antibodies -online | ABIN6127 745 | | Rabbit | Human | | WB, IF | | Unconj . | Poly. |
| Abbexa Ltd | abx10024 5 | | Rabbit | Human | | WB, IHC, ICC, IP | aa 18-110 | Unconj. | Poly. |
| Bioss Inc. | bs-12996R | Protein A purified | Rabbit | Human | IgG | WB, ELISA, Flo, ICC, IF, IHC-fr, IHC-p | | Unconj. | Poly. |
| Bioss Inc. | bs-12996R-Biotin | Protein A purified | Rabbit | Human | IgG | WB, ELISA, IHC-p, IHC-fr | | Biotin | Poly. |
| Bioss Inc. | bs-12996R-Cy3 | Protein A purified | Rabbit | Human | IgG | Flo, ICC, IF, IHC-fr, IHC-p | | Cy3 | Poly. |
| Bioss Inc. | bs-12996R-Cy5 | Protein A purified | Rabbit | Human | IgG | Flo, IHC-p, IHC-fr, ICC | | Cy5 | Poly. |
| Bioss Inc. | bs-12996R-Cy5.5 | Protein A purified | Rabbit | Human | IgG | Flo, IHC-p, IHC-fr, ICC | | Cy5.5 | Poly. |
| Bioss Inc. | bs-12996R-Cy7 | Protein A purified | Rabbit | Human | IgG | Flo, IHC-p, IHC-fr, ICC | | Cy7 | Poly. |
| Immundia gnostik AG | AE1065.1 , AE1065.2 | Serum | Rabbit | Human | | ELISA, RIA | aa 66-80 | Unconj. | Poly. |
| Immundia gnostik AG | AE1061.1 , AE1061.2 | Serum | Rabbit | Human | | ELISA, WB | aa 96-110 | Unconj. | Poly. |
| Santa Cruz Biotechno logy, Inc. | sc-393728 | | Mouse | Human | | WB, IP, IF, ELISA | E-7 | Unconj. | Mono. |
| Santa Cruz Biotechno logy, Inc. | sc-33656 | | Mouse | Mouse, Rat, Human | | WB, IP, IF, IHC-p | G-81 | Unconj. | Mono. |
| Santa Cruz Biotechno logy, Inc. | sc-398429 | | Mouse | Human | | WB, IP, IF, ELISA | H-12 | Unconj. | Mono. |
| OriGene Technolo gies | AP17270 PU-N | Saturated Ammonium Sulfate (SAS) precipitation followed by dialysis against PBS | Rabbit | Human | | WB | C-termin al | Unconj. | Poly. |
| OriGene Technolo gies | AP02089 SU-N, AP02089 SU-S | Serum | Rabbit | Human | | ELISA, RIA | aa 66-80 | Unconj. | Poly. |
| OriGene Technologies | AP02090 SU-N, AP02090 SU-S | Serum | Rabbit | Human | | ELISA, WB | aa 96-110 | Unconj. | Poly. |
| OriGene Technolo gies | TA81172 0S | Affinity chromatogra phy (protein A/G) | Mouse | Human | IgG1 | WB | aa 20-110 | Unconj. | Mono. |
| Hycult Biotech | HM2040 | | Mouse | Human | IgM | WB, IA, IHC-p | G-81 | Unconj. | Mono. |
| United States Biological | 313376-50ug, 313376-100ug | Purified by Protein G affinity chromatogra phy | Rabbit | Human | | ELISA, WB | | Unconj. | Poly. |
| United States Biological | D3213-01B-20ul, D3213-01B-100ul | Serum | Rabbit | Human | IgG | ELISA, WB | aa 96-110 | Unconj. | Poly. |
| United States Biological | 313377-50ug, 313377-100ug | Purified by Protein G affinity chromatogra phy | Rabbit | Human | | ELISA, WB | | Biotin | Poly. |
| ProSci, Inc | 62-426 | Saturated Ammonium Sulfate (SAS) precipitation followed by dialysis | Rabbit | Human | Ig | WB, Flo, IHC-p | aa 74-103 | Unconj. | Poly. |
| GeneTex | GTX5558 5 | Purified by affinity chromatogra phy | Rabbit | Mouse, Human | IgG | WB, ICC, IF | aa 20-110 | Unconj. | Poly. |
| GeneTex | GTX5442 8 | Unpurified | Mouse | Human | IgM | WB, ELISA, IHC-p | G-81 | Unconj. | Mono. |
| GeneTex | GTX8175 6 | Saturated Ammonium Sulfate (SAS) precipitation followed by dialysis against PBS | Rabbit | Human | IgG | WB, Flo, IHC-p | aa 74-103 | Unconj. | Poly. |
| Abcam | ab175519 | Saturated Ammonium Sulfate (SAS) precipitation followed by dialysis against PBS | Rabbit | Human | IgG | WB, FCM, IHC-p | | Unconj. | Poly. |
| RayBiotec h | 144-07280-50, 144-07280-100, 144-07280-200 | Affinity purification | Rabbit | Mouse, Human | IgG | WB, IF | | Unconj. | Poly. |
| RayBiotec h | 102-16906 | Affinity purification | Rabbit | Human | Ig | WB, Flo, IHC-p | aa 74-103 | Unconj. | Poly. |
| EpiGente k | A71168 | Affinity purification | Rabbit | Mouse, Human | IgG | WB, IF | aa 20-110 | Unconj. | Poly. |
| Thermo Fisher Scientific | PA5-96623 | Antigen affinity chromatogra phy | Rabbit | Mouse, Human | IgG | ICC, WB | aa 20-110 | Unconj. | Poly. |
| Thermo Fisher Scientific | PA5-77187 | Antigen affinity chromatogra phy | Rabbit | Mouse, Human | IgG | ICC, WB | full length | Unconj. | Poly. |
| Thermo Fisher Scientific | PA5-66649 | Antigen affinity chromatogra phy | Rabbit | Human | IgG | IHC | | Unconj. | Poly. |
| ABclonal Technolo gy | A7280 | Affinity purification | Rabbit | Mouse, Human | IgG | WB, IF | aa 20-110 | Unconj. | Poly. |
| Abnova Corporati on | H001171 59-W01P | | Rabbit | Human | | WB, Flo, IF | full length | Unconj. | Poly. |
| Wuhan Fine Biotech Co., Ltd. | FNab022 63 | Affinity purified, ≥95% as determined by SDS-PAGE | Rabbit | Human | IgG | ELISA, IHC | | Unconj. | Poly. |
| Creative Biolabs | CBMAB-D0335-YC | | Mouse | Human | IgM | WB, ELISA, IHC | 13A43 | Unconj. | Mono. |
| Creative Biolabs | CBMAB-X0125-FY | | Mouse | Human | IgM | WB, IA, IHC-p | CBFY A-0090 | Unconj. | Mono. |
| Creative Biolabs | CBMAB-H0984-FY | | Mouse | Human | IgM | WB, ELISA, IHC-p | CBFY H-0123 | Unconj. | Mono. |
| Creative Biolabs | CBMAB-D0336-YC | | Mouse | Human | IgM | WB, ELISA, IHC-p | G-81 | Unconj. | Mono. |
| Creative Biolabs | CBMAB-D0337-YC | | Mouse | Human | IgM | WB, ELISA, IHC-p | GF1 | Unconj. | Mono. |
| Creative Biolabs | MOB-3969z | Purity>95% by SDS-PAGE | Mouse | Human | IgG1 | WB, ELISA, IF, FA | 33B8 | Unconj. | Mono. |
| Creative Diagn. | DPABH-11629 | | Rabbit | Human | IgG | WB, Flo, IHC-p | aa 81-110 | Unconj. | Poly. |
| Creative Diagn. | DCABH-11224 | | Rabbit | Human | IgG | WB, ELISA | | Unconj. | Mono. |
| Creative Diagn. | CABT-B8854 | | Mouse | Human | IgM | WB, IA, IHC-p | H92 | Unconj. | Mono. |

Also preferably, the determination of the peptide's concentration is performed by measuring the levels of gene expression encoding for a peptide comprising a sequence SEQ ID No.: 17 (which is fragmented subsequently into any of the peptides of SEQ ID Nos.: 1 to 16), in an ex vivo biological sample, via a polymerase chain reaction (PCR)-based assay. In a preferred embodiment of the invention said PCR-based assay includes the use of a colorimetric or a fluorometric diagnostic strip comprising a DNA probe, or nanoparticles comprising a DNA probe. More preferably said PCR-based assay is a RT-qPCR.

Another embodiment of the present invention relates to a kit for determining *in vitro* the level of resistance of a healthy individual to suffer from symptoms of a virus affecting the respiratory tract, wherein said kit comprises:
- means for storing an ex *vivo* biological sample of said healthy individual selected from the group consisting of a nasopharyngeal sample, a blood sample, a plasma sample, a urine sample, a human milk sample, a sweat sample and a teardrop sample;
- means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, as disclosed in table 1 herein, in said stored *ex vivo* biological sample.

As indicated previously in this disclosure, once dermcidin (SEQ ID No.: 17) is secreted, is processed or fragmented to give rise to different dermcidin fragments of SEQ ID Nos.: 1 to 16 described in table 1 of present description.

Additionally, also a previously indicated, the presence of dermcidin and of any of the fragments thereof, as disclosed in table 1 is significantly higher in ex *vivo* biological samples from healthy individuals who have never suffered from influenza symptoms, when compared to the values of healthy individuals sensitive or prone to having respiratory tract viral infections (figure 1). On the other hand, present invention also shows that the production of these peptides (dermcidin, i.e., a peptide of SEQ ID No.: 17 and any fragments thereof of SEQ ID No.: 1 to 16) is stimulated in response to infection by a respiratory virus (see figure 2).

Accordingly, this may be used to determine the group of healthy individuals who are more prone to suffer said disease. This may be of particular interest in determining the groups of healthy individuals for whom, the use of prophylactic measures, such as vaccination against said viral disease, may be recommended.

To that regard, another aspect of present invention refers to an *in vitro* method for selecting healthy individuals in need of prophylactic measures against a disease caused by a virus affecting the respiratory tract, wherein said method comprises:
- determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14,15 and 16, as disclosed in table 1 herein, in an ex *vivo* biological sample of a healthy individual selected from the group consisting of a saliva sample, a nasopharyngeal sample, a blood sample, a plasma sample, a urine sample, a human milk sample, a sweat sample and a teardrop sample;
- comparing the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16 of said ex *vivo* biological sample with a threshold value; and
- selecting said healthy individual for prophylactic measures against said disease if the value of said total concentration of said peptides in the ex *vivo* biological sample of said individual is lower than said threshold value.

Another additional aspect of present invention refers to a kit for selecting healthy individuals in need of prophylactic measures against a disease caused by a virus affecting the respiratory tract, wherein said kit comprises:
- means for storing an ex *vivo* biological sample of a healthy individual selected from the group consisting of a saliva sample, a nasopharyngeal sample, a blood sample, a plasma sample, a urine sample, a human milk sample, a sweat sample and a teardrop sample;
- means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in said stored ex *vivo* biological sample;
- means and/or instructions for identifying values of said total concentration of peptides which are lower of a threshold value.

In a preferred embodiment of the invention said prophylactic measures include but are not limited to respiratory masks (for nose and mouth), including hygienic masks, surgical masks, FFP2 masks, N95 masks, KN95 masks, FFP3 masks, among others; individual medical protective equipment including gloves, protective glasses and lab coats or gowns among others; preventive isolation, social distancing, hand wash or sanitization, vaccines against a virus affecting the respiratory tract, nutraceuticals protecting or increasing the individual protection against a virus affecting the respiratory tract and preventive medicaments against a virus affecting the respiratory tract.

Accordingly, present invention refers to an *in vitro* method and to a kit for conducting said method, for selecting healthy individuals in need of prophylactic measures against a disease caused by a virus affecting the respiratory tract, as described herein, wherein said prophylactic measures are selected from the group consisting of a respiratory mask, gloves, protective glasses, a lab coat, preventive isolation, social distancing, vaccines against a virus affecting the respiratory tract, nutraceuticals protecting or increasing the individual protection against a virus affecting the respiratory tract and preventive medicaments against a virus affecting the respiratory tract.

Thus, one embodiment of present invention refers to an *in vitro* method for selecting healthy individuals in need of being vaccinated against a disease caused by a virus affecting the respiratory tract, wherein said method comprises:
- determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14,15 and 16, as disclosed in table 1 herein, in an ex *vivo* biological sample of a healthy individual selected from the group consisting of a saliva sample, a nasopharyngeal sample, a blood sample, a plasma sample, a urine sample, a human milk sample, a sweat sample and a teardrop sample;
- comparing the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16 of said ex *vivo* biological sample with a threshold value; and
- selecting said healthy individual for being vaccinated against said disease if the value of said total concentration of said peptides in the ex *vivo* biological sample of said individual is lower than said threshold value.

Another embodiment of present invention refers to an *in vitro* method for selecting healthy individuals in need of a preventive medicament against a disease caused by a virus affecting the respiratory tract, wherein said method comprises:
- determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14,15 and 16, as disclosed in table 1 herein, in an ex *vivo* biological sample of a healthy individual selected from the group consisting of a saliva sample, a nasopharyngeal sample, a blood sample, a plasma sample, a urine sample, a human milk sample, a sweat sample and a teardrop sample;
- comparing the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16 of said ex *vivo* biological sample with a threshold value; and
- selecting said healthy individual for preventive treatment with a medicament against said disease if the value of said total concentration of said peptides in the ex *vivo* biological sample of said individual is lower than said threshold value.

Yet another embodiment of present invention refers to an *in vitro* method for selecting healthy individuals in need of a preventive nutraceutical against a disease caused by a virus affecting the respiratory tract, wherein said method comprises:
- determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14,15 and 16, as disclosed in table 1 herein, in an ex *vivo* biological sample of a healthy individual selected from the group consisting of a saliva sample, a nasopharyngeal sample, a blood sample, a plasma sample, a urine sample, a human milk sample, a sweat sample and a teardrop sample;
- comparing the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16 of said ex *vivo* biological sample with a threshold value; and
- selecting said healthy individual for preventive treatment with a nutraceutical against said disease if the value of said total concentration of said peptides in the ex *vivo* biological sample of said individual is lower than said threshold value.

Yet another embodiment of present invention refers to a kit for selecting healthy individuals in need of prophylactic measures against a disease caused by a virus affecting the respiratory tract, as described herein, wherein said prophylactic measures are selected from the group consisting of a respiratory mask, gloves, protective glasses, a lab coat, preventive isolation, social distancing, vaccines against a virus affecting the respiratory tract, nutraceuticals protecting or increasing the individual protection against a virus affecting the respiratory tract and preventive medicaments against a virus affecting the respiratory tract, and wherein said kit comprises:
- means for storing an ex *vivo* biological sample of a healthy individual selected from the group consisting of a saliva sample, a nasopharyngeal sample, a blood sample, a plasma sample, a urine sample, a human milk sample, a sweat sample and a teardrop sample;
- means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in said stored ex *vivo* biological sample;
- means and/or instructions for identifying values of said total concentration of peptides of said ex *vivo* biological sample which are lower of a threshold value.

One additional embodiment of present invention refers to a method for vaccinating a healthy individual in need thereof, against a disease caused by a virus affecting the respiratory tract, wherein said method comprises:
- determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14,15 and 16, as disclosed in table 1 herein, in an ex *vivo* biological sample of said healthy individual selected from the group consisting of a saliva sample, a nasopharyngeal sample, a blood sample, a plasma sample, a urine sample, a human milk sample, a sweat sample and a teardrop sample;
- comparing the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16 of said ex *vivo* biological sample with a threshold value; and
- administering a vaccine against said disease to said healthy individual when the total concentration value in the ex *vivo* biological sample of said individual is lower than said threshold value.

Another additional embodiment of present invention refers to a method of treating a healthy individual in need thereof with a preventive medicament against a disease caused by a virus affecting the respiratory tract, wherein said method comprises:
- determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14,15 and 16, as disclosed in table 1 herein, in an ex *vivo* biological sample of said healthy individual selected from the group consisting of a saliva sample, a nasopharyngeal sample, a blood sample, a plasma sample, a urine sample, a human milk sample, a sweat sample and a teardrop sample;
- comparing the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16 of said ex *vivo* biological sample with a threshold value; and
- administering the preventive medicament against said disease to said healthy individual when the total concentration value in the ex *vivo* biological sample of said individual is lower than said threshold value.

Another additional embodiment of present invention refers to a method of treating a healthy individual in need thereof with a preventive nutraceutical against a disease caused by a virus affecting the respiratory tract, wherein said method comprises:
- determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14,15 and 16, as disclosed in table 1 herein, in an ex *vivo* biological sample of said healthy individual selected from the group consisting of a saliva sample, a nasopharyngeal sample, a blood sample, a plasma sample, a urine sample, a human milk sample, a sweat sample and a teardrop sample;
- comparing the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16 of said ex *vivo* biological sample with a threshold value; and
- administering the preventive nutraceutical against said disease to said healthy individual when the total concentration value in the ex *vivo* biological sample of said individual is lower than said threshold value.

Another embodiment of present invention refers to a kit for vaccinating healthy individuals in need thereof against a disease caused by a virus affecting the respiratory tract, wherein said kit comprises:
- means for storing an ex *vivo* biological sample of a healthy individual selected from the group consisting of a saliva sample, a nasopharyngeal sample, a blood sample, a plasma sample, a urine sample, a human milk sample, a sweat sample and a teardrop sample;
- means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in said stored ex *vivo* biological sample;
- means for identifying values of said total concentration of peptides which are lower of a threshold value, and
- means and/or instructions for vaccinating said healthy individual against said disease.

Yet another embodiment of present invention refers to a kit for treating healthy individuals in need thereof with a preventive medicament against a disease caused by a virus affecting the respiratory tract, wherein said kit comprises:
- means for storing an ex *vivo* biological sample of a healthy individual selected from the group consisting of a saliva sample, a nasopharyngeal sample, a blood sample, a plasma sample, a urine sample, a human milk sample, a sweat sample and a teardrop sample;
- means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in said stored ex *vivo* biological sample;
- means for identifying values of said total concentration of peptides which are lower of a threshold value, and
- means and/or instructions for administering said preventive medicament against the disease to said healthy individual.

Yet another embodiment of present invention refers to a kit for treating healthy individuals in need thereof with a preventive nutraceutical against a disease caused by a virus affecting the respiratory tract, wherein said kit comprises:
- means for storing an ex *vivo* biological sample of a healthy individual selected from the group consisting of a saliva sample, a nasopharyngeal sample, a blood sample, a plasma sample, a urine sample, a human milk sample, a sweat sample and a teardrop sample;
- means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in said stored ex *vivo* biological sample;
- means for identifying values of said total concentration of peptides which are lower of a threshold value, and
- means and/or instructions for administering said preventive nutraceutical against the disease to said healthy individual.

In an embodiment, said ex *vivo* biological sample is selected from the group consisting of a saliva sample, a nasopharyngeal sample, a blood sample, a plasma sample, a urine sample, a placenta sample, a human milk sample, a sweat sample and a teardrop sample.

Preferably, said ex *vivo* biological sample is selected from the group consisting of a saliva sample, a nasopharyngeal sample and a teardrop sample. Even more preferably said ex *vivo* biological sample is a nasopharyngeal sample.

Preferably the means for storing the ex *vivo* biological sample may be selected from the group consisting of a vial, a nasopharyngeal swab, a container, a pasteur pipette, a bottle, a plate and a tube, among others.

The means for storing the ex *vivo* biological sample are, sterile or sterilized and inert, i.e., do not interact or react with the peptides to be measured in the ex *vivo* biological sample during storage. Said means for storing the ex *vivo* biological sample may be single use or reusable. Said means for storing the ex *vivo* biological samples are adequate to keep the sample up to the moment in which the determination of the total concentration of peptides is carried out, which may be immediately after the sample is provided, or may also include a storage period (for example, including cold storage or cryopreservation).

In an embodiment of the invention the means for determining the total concentration of peptides include any means which may be used in a method which is capable to measure the concentration of a protein or a peptide comprising any of sequences SEQ ID Nos. 1 to 17 in a biological sample, and in particular said means for determining the total concentration of peptides are means to conduct mass spectrometry (MS), liquid chromatography-mass spectrometry (LC-MS), high pressure liquid chromatography (HLPC), immunoassay and polymerase chain reaction (PCR)-based assays.

In a preferred embodiment of the invention the means for determining the total concentration of peptides are means to conduct an immunoassay. Preferably said means for determining the total concentration of peptides are means to conduct an ELISA test, an ELISPOT test, a bead-based multiplex immunoassay (Immunoflex), a Western Blot assay, a flow cytometry assay, a fluorescence-activated cell sorting assay (FACS), a radioimmunoassay, a colorimetric or a fluorometric diagnostic strip comprising antibodies, colorimetric nanoparticles comprising antibodies, a chemiluminescent assay, an immunocytochemistry assay (with or without paraffin protocol), an immunofluorescence assay, an immunohistochemical assay, among others.

Said antibodies may be monoclonal antibodies or polyclonal antibodies. Preferably said antibodies are an Ig, an IgG, an IgG1 or an IgM isotype. In some preferred embodiments said antibodies are unconjugated, in other preferred embodiments said antibodies are conjugated or tagged with HRP (horse radish peroxidase), FITC (fluorescein isothiocyanate), Biotin or with cyanine dyes such as Cy3, Cy5, Cy5.5 or Cy7.

Examples of antibodies specific for binding to a peptide comprising any of sequences SEQ ID Nos. 1 to 17 are those included in table 1.3 included in present disclosure.

In another preferred embodiment the means determining the total concentration of peptides are means to measure the levels of gene expression encoding for a peptide comprising a sequence SEQ ID No.: 17 (which is fragmented subsequently into any of the peptides of SEQ ID Nos.: 1 to 16), in an ex vivo biological sample, with a PCR-based assay. More preferably, said PCR-based assay is a colorimetric or a fluorometric diagnostic strip comprising a DNA probe, or nanoparticles comprising a DNA probe. Even more preferably said PCR-based assay is a RT-qPCR assay.

In a preferred embodiment the means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in the ex *vivo* biological sample, included in the kit of present invention, include antibodies specific for binding to a peptide comprising any of sequences SEQ ID Nos. 1 to 17, as disclosed in table 1 herein.

The means for identifying values of said total concentration of peptides may be any means that allows the user of the kit to separate the ex *vivo* biological samples having a total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, which are lower than a certain threshold value.

According to present invention said threshold value is measured in the same type of ex *vivo* biological sample and using the same method to determine the concentration of peptides, as in the individual to which is compared to in the method and kits of the invention and represents a value lower than the average concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16 in a group of individuals who have never suffered from influenza symptoms. In a preferred embodiment said threshold value is lower, more preferably 5%, 15%, 25%, 50%, 80%, 99% lower, than the average concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 1 6 in a group of individuals who never suffered from influenza symptoms.

In a preferred embodiment said means for identifying values of said total concentration of peptides which are lower than a certain threshold value are colorimetric means, fluorometric means, spectrophotometric means or reactive means comprising a compound or composition which reacts with said peptides to form a detectable form at concentrations higher than said certain threshold value.

In some cases, the means for vaccinating the individuals according to present invention may comprise only the vaccine itself. In other cases, the means for vaccinating the individuals according to present invention may comprise both a container to administer the vaccine and the vaccine itself. In an embodiment the vaccine is an injectable vaccine and the means for vaccinating the individuals comprise a vaccine container and a syringe. In another embodiment the vaccine is a for nasal administration and the means for vaccinating the individuals comprise a vaccine container and applier for nasal administration.

In some cases, the means for treating the individuals with a preventive medicament, or a preventive nutraceutical against the disease caused by a virus of the respiratory tract may comprise only the preventive medicament or nutraceutical. In other cases, the means for treating the individuals with a preventive medicament or nutraceutical against the disease caused by a virus of the respiratory tract may comprise both a container to administer the preventive medicament or nutraceutical and the preventive medicament or nutraceutical itself.

In an embodiment disclosed herein the preventive medicament is an oral medicament or nutraceutical, an injectable medicament or a nasal medicament or nutraceutical. In another embodiment the preventive medicament is an injectable drug and the means for administering the medicament comprise a syringe. In another embodiment the preventive medicament or nutraceutical is for nasal administration and the means for administering the medicament or nutraceutical comprise a container and an applier for nasal administration. In yet another embodiment the preventive medicament or nutraceutical is for oral administration and the means for administering the medicament or nutraceutical comprise a container and the oral preventive medicament or nutraceutical which may be a tablet, a pill or a capsule. In yet another embodiment the preventive medicament or nutraceutical are eye drops. In yet another embodiment the preventive medicament or nutraceutical is for skin administration and the means for administering the medicament or nutraceutical comprise wipes containing said preventive medicament or nutraceutical.

In one embodiment, the preventive medicament is a preventive medicament or nutraceutical against an influenza virus; a coronavirus; a syncytial virus (RSV); a rhinovirus (RVs); a parainfluenza virus (PIV); a metapneumovirus; a bocavirus; an adenovirus (ADV); and a morbillivirus, such as a measles virus. Preferably, said vaccine is a vaccine against an influenza virus (flu virus), a coronavirus HcoV-OC43 (common cold virus), SARS, MERS, SARS-CoV-2, a syncytial virus (RSV); a rhinovirus (RVs); a parainfluenza virus (PIV); an adenovirus (ADV); or a morbillivirus.

Preferably said preventive medicament is a bronchodilator, an antiviral, a probiotic medicament, oxygen, nitric oxide, N-acetyl-cysteine, or a medicament to stimulate the immune system, among others. More preferably, said preventive medicament is a peptide comprising or consisting of a sequence SEQ ID No.: 1, and even more preferably, said preventive medicament is a peptide comprising or consisting of a sequence SEQ ID No.: 1, 2, 3, 4, 5, 6, 7, 8 and 17; or a sequence selected from the group consisting of SEQ ID Nos.: 19, 20, 21, 22, 23, 24, 25, 26 and 32, or a derivative thereof.

Preferably, said preventive nutraceutical is vitamin C, vitamin D, calendula, propolis, echinacea, elderberry, ginseng, honey, thyme, lemon, ginger, umeboshi, and derivatives thereof; a probiotic or a nutraceutical to stimulate the immune system, among others. More preferably, said preventive nutraceutical is a peptide comprising or consisting of a sequence SEQ ID No.: 1, and even more preferably, said preventive nutraceutical is a peptide comprising or consisting of a sequence SEQ ID No.: 1, 2, 3, 4, 5, 6, 7, 8 and 17; or a sequence selected from the group consisting of SEQ ID Nos.: 19, 20, 21, 22, 23, 24, 25, 26 and 32, or a derivative thereof.

In an embodiment the vaccine is a vaccine against an influenza virus; a coronavirus; a syncytial virus (RSV); a rhinovirus (RVs); a parainfluenza virus (PIV); a metapneumovirus; a bocavirus; an adenovirus (ADV); and a morbillivirus, such as a measles virus. Preferably, said vaccine is a vaccine against an influenza virus (flu virus), a coronavirus HcoV-OC43 (common cold virus), SARS, MERS, SARS-CoV-2, a syncytial virus (RSV); a rhinovirus (RVs); a parainfluenza virus (PIV); an adenovirus (ADV); or a morbillivirus.

More preferably, said vaccine is a vaccine against an influenza virus (flu virus), a coronavirus HcoV-OC43 (common cold virus), SARS, MERS or SARS-CoV-2.

In an even preferred embodiment the vaccine is a vaccine against an influenza virus, a coronavirus or a morbillivirus. In a more preferred embodiment, the coronavirus is HcoV-OC43 or SARS-CoV-2, the morbillivirus is a measles virus and the influenza virus comprises an H1N1 subtype and/or an H3N2 subtype.

### EXAMPLES

The examples described below are for purposes of illustration only and are not meant to limit the scope of the present invention.

### Example 1: Method for the in vitro quantification of dermcidin content in ex vivo biological samples

The protocol recommended in the Human Proteolysis Inducing Factor/Dermcidin (PIF/DCD) ELISA Kit (catalogue number: CSB-E13626h) of CUSABIO BIOTECH CO., LTD, was modified to detect dermcidin in human nasopharyngeal swab samples. The original kit used determines the concentration of dermcidin and human proteolysis-inducing factor (PIF) in plasma, serum, cell culture supernatants, urine, and tissue homogenates, but not in nasopharyngeal swab samples, teardrop samples or oral mucosa samples. Based on this kit, a method has been developed using the above-mentioned kit for the quantification of dermcidin also in nasopharyngeal swab samples, teardrop samples and oral mucosa samples.

The method and kit of the invention uses a sandwich assay employing antibodies that coat the test wells specific for any of peptides of SEQ ID Nos.: 1 to 17, i.e. specific for dermcidin (SEQ ID No. 17) or any fragment thereof (SEQ ID Nos.: 1 to 16) as disclosed in table 1.

Modifying the kit recommendations, the pre-processing of samples for the detection of dermcidin (including any fragment thereof) is as follows:
- The nasopharyngeal swab samples were collected either in Copan Diagnostics Universal Transport Medium (UTM-RT^{™}) System or in biocomma^{®} Virus Transport and was submerged in a sampling tube with 3 ml of Preservation Medium and stored at -80°C since collection until used. For the analysis, sampling tubes were thawed on ice, vortexed during 5-10 seconds and 200 µl of the resulting liquid was used directly to start the ELISA measurement.
- The teardrop samples were collected with a pipette from the inferior eyelid and stored at - 80°C since collection until used. For the analysis the samples were thawed on ice and diluted with the sample buffer up to the 200µl required to conduct the analysis.
- The oral mucosa samples were taken with a swab by rubbing it against the interior of the cheek (3 times each side), avoiding touching any other mouth tissue (tongue, teeth, gums, etc.). The swab was then submerged in a vial with 1 ml PBS and frozen at -80°C until the analysis was conducted. For the analysis the sample was thawed on ice, vortexed during 30 seconds, and subsequently rubbed against the walls of the vial to release all the adhered material and a 200 µl liquid aliquot was used to conduct the analysis.

With this method it was assessed the total concentration of dermcidin and of any fragment thereof (i.e. the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16) in nasopharyngeal swab samples, teardrop samples and oral mucosa samples from healthy individuals who are sensitive or prone to having respiratory tract infection symptoms and also, from healthy individuals who never suffered from influenza symptoms as well as samples from individuals hospitalized for respiratory tract pathology and positive by nasopharyngeal PCR test for the following respiratory viruses: influenza A virus, respiratory syncytial virus (RSV), metapneumovirus, parainfluenza virus (PIV), rhinovirus (RV), adenovirus (ADV), common coronavirus or bocavirus.

As shown in figure 1, the levels measured in nasopharyngeal samples show that people who never has suffered from flu symptoms have over 5 times more dermcidin in their nasopharynx than the sensitive ones (mean values of 71,78 ng/ml for asymptomatic compared to 13,08 ng/ml for sensitives).

Moreover, as shown in figure 2, when people are infected with influenza ("Influenza" in figure 2) or any other respiratory virus such as respiratory syncytial virus (RSV), metapneumovirus, parainfluenza virus (PIV), rhinovirus (RV), adenovirus (ADV), common coronavirus or bocavirus ("Other" in figure 2), their immune system responds over-segregating dermcidin in their nasopharyngeal fluid (figure 2).

Besides, it could be confirmed that the levels of dermcidin (measured as the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16) could also be measured in other human fluids of healthy individuals like tears (figure 3).

It can be seen that not all types of biological samples are suited for conducting the methods disclosed in present invention. For example oral mucosa samples appear not to be adequate to conduct the *in vitro* methods of present invention as seen in figure 3, due to the low levels of dermcidin peptides present in the oral mucosa.

## Claims

1. An *in vitro* method for determining the level of resistance of a healthy individual to suffer from symptoms of a virus affecting the respiratory tract, wherein said method comprises determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in an ex *vivo* biological sample of a healthy individual selected from the group consisting of a nasopharyngeal sample, a blood sample, a plasma sample, a urine sample, a human milk sample, a sweat sample and a teardrop sample; wherein the higher the concentration of said peptides in the sample is, the greater the resistance of said individual is to suffer from symptoms of said virus.

2. An *in vitro* method of selecting healthy individuals in need of prophylactic measures against a disease caused by a virus affecting the respiratory tract, wherein said method comprises:
- determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in an *ex vivo* biological sample of a healthy individual selected from the group consisting of a saliva sample, a nasopharyngeal sample, a blood sample, a plasma sample, a urine sample, a human milk sample, a sweat sample and a teardrop sample;
- comparing said total concentration of peptides in said ex *vivo* biological sample with a threshold value; and
- selecting said healthy individual in need of prophylactic measures against said disease if the value of said total concentration of said peptides in the ex *vivo* biological sample of said individual is lower than said threshold value.

3. The *in vitro* method according to claim 2, wherein said prophylactic measures are selected from the group consisting of a respiratory mask, gloves, protective glasses, a lab coat, a nutraceutical protecting or increasing the individual protection against a virus affecting the respiratory tract, a preventive medicament against said virus and a vaccine against said virus.

4. The *in vitro* methods according to any of claims 1 to 3, wherein the ex *vivo* biological sample is a nasopharyngeal sample.

5. The *in vitro* methods according to any of claims 1 to 4, wherein the virus affecting the respiratory tract is selected from the group consisting of: an influenza virus; a coronavirus; a syncytial virus (RSV); a rhinovirus (RVs); a parainfluenza virus (PIV); an adenovirus (ADV); and a morbillivirus, such as a measles virus.

6. The *in vitro* methods according to any of claims 1 to 5, wherein the virus affecting the respiratory tract is selected an influenza virus, a coronavirus HcoV-OC43, SARS, MERS and SARS-CoV-2.

7. The *in vitro* methods according to any of claims 1 to 6, wherein the virus affecting the respiratory tract is SARS-CoV-2.

8. The *in vitro* methods according to any of claims 1 to 6, wherein the virus affecting the respiratory tract is an influenza virus.

9. The *in vitro* methods according to any of claims 1 to 8, wherein the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16 is determined by immunoassay comprising antibodies specific for binding to a peptide comprising any of sequences SEQ ID Nos. 1 to 17.

10. The *in vitro* methods according to any of claims 1 to 8, wherein the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16 is determined by a PCR-based assay.

11. A kit for conducting the method for determining the level of resistance of a healthy individual to suffer from symptoms of a virus affecting the respiratory tract, according to claim 1 or any of claims 4 to 10, wherein said kit comprises:
- means for storing an ex *vivo* biological sample of a healthy individual selected from the group consisting of a nasopharyngeal sample, a blood sample, a plasma sample, a urine sample, human milk sample, a sweat sample and a teardrop sample;
- means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in said stored ex *vivo* biological sample.

12. A kit for conducting the method of selecting healthy individuals in need of prophylactic measures against a disease caused by a virus affecting the respiratory tract, according to any of claims 2 to 10, wherein said kit comprises:
- means for storing an ex *vivo* biological sample of a healthy individual selected from the group consisting of a saliva sample, a nasopharyngeal sample, a blood sample, a plasma sample, a urine sample, a human milk sample, a sweat sample and a teardrop sample;
- means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16, in said stored ex *vivo* biological sample;
- means and/or instructions for identifying values of said total concentration of peptides which are lower of a threshold value.

13. The kit according to any of claims 11 or 12, wherein the means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16 is an immunoassay comprising antibodies specific for binding to a peptide comprising any of sequences SEQ ID Nos. 1 to 17.

14. The kit according to any of claims 11 or 12, wherein the means for determining the total concentration of peptides comprising a sequence SEQ ID No.: 17 and of any fragment thereof having a sequence selected from the group consisting of SEQ ID Nos.: 1, 2, 3, 4, 5, 6, 7, 8, 9, 10, 11, 12, 13, 14, 15 and 16 is a PCR-based assay.

15. The kit according to any of claims 11 to 14, wherein the means to store the ex *vivo* biological sample is a nasopharyngeal swab.
